# EUROPEAN PATENT APPLICATION

(11) **EP 4 603 063 A1**
(43) Date of publication of application: **20.08.2025**
(21) Application number: 22954177.6
(22) Date of filing: 28.12.2022
(51) Int. Cl.: A61F 2/32

(54) **ARTIFICIAL HIP JOINT PROSTHESIS AND MOUNTING METHOD**

(30) Priority: 13.10.2022 CN 202211255078
(71) Applicant: Beijing Naton Medical Technology Holdings Co., Ltd., Beijing 100094 (CN)
(72) Inventor: XU, Hui, Beijing 100094 (CN); WANG, Meng, Beijing 100094 (CN); HU, Xiaoqiang, Beijing 100094 (CN)
(74) Representative: advotec.
(86) International application number: PCT/CN2022/142956
(87) International publication number: WO 2024/077790

(57) **Abstract**

An artificial hip joint prosthesis and a method for mounting the same are provided. The artificial hip joint prosthesis includes a spherical chamber and a ball head. An outer spherical surface of the ball head fits with an inner spherical surface of the spherical chamber to form a spherical hinge. An inner spherical surface of the spherical chamber has an opening in an elliptical shape or a similar shape with a long axis and a short axis, to allow insertion of the ball head. The long axis of the opening in the elliptical shape or the similar shape has a length A, and the short axis of the opening has a length B. The ball head is mounted inside the spherical chamber and has a diameter C, in which B < A ≤ C.

## Description

### CROSS-REFERENCE TO RELATED APPLICATION

This application claims priority to and benefits of Chinese Patent Application Serial No. 2022112550789, filed on October 13, 2022, the entire content of which is incorporated herein by reference.

### FIELD

The present invention relates to the field of medical prosthesis, and more particularly to an artificial hip joint prosthesis.

### BACKGROUND

A human hip joint is composed of acetabulum and femoral head that is embedded in the acetabulum and is rotatable to form the hip joint. Artificial hip replacement surgery is a method of treating the hip joint by replacing the human femoral head with a femoral ball-head prosthesis and the human acetabulum with an acetabular cup prosthesis. The design of artificial hip joints often encounter problems that a femoral head prosthesis tends to come out of an acetabular prosthesis or a ball-head prosthesis tends to come out of a spherical chamber prosthesis. Two solutions to the above problems are commonly adopted. One of the solutions is to make a diameter of an entrance of the spherical chamber prosthesis slightly smaller than a diameter of the ball-head prosthesis, and force the ball head into the spherical chamber by using instruments and by deformation of the entrance of the spherical chamber prosthesis during insertion of the ball head into the spherical chamber. This solution is simple in structure but has disadvantages of longer operation process and more instruments. Moreover, since the diameter of the entrance of the spherical chamber cannot be much smaller than the diameter of the ball head, which may otherwise have difficulty in pressing the ball head into the chamber, a force preventing the ball head from detaching from the spherical chamber is limited. The other solution is to make the spherical chamber and the ball head equal in diameter, and mount a snap ring at the entrance of the spherical chamber after the ball head is mounted into the chamber to prevent the detachment of the ball head. This solution can meet requirements of easy insertion and anti-detachment of the ball head, but the mounting of the snap ring prolongs the surgical process and time. Moreover, the additional locking ring and a groove or a similar structure for mounting the locking ring at the entrance of the spherical chamber add to the complexity of the prosthesis.

### SUMMARY

Embodiments of the present invention propose a hip joint prosthesis that can prevent a ball head from detaching from an inner liner and improve safety of using the hip joint prosthesis.

In one aspect, embodiments of the present invention provide an artificial hip joint prosthesis including a spherical chamber and a ball head. An outer spherical surface of the ball head fits with an inner spherical surface of the spherical chamber to form a spherical hinge. An inner spherical surface of the spherical chamber has an opening in an elliptical shape or a similar shape with a long axis and a short axis, to allow insertion of the ball head. The long axis of the opening in the elliptical shape or the similar shape has a length A, and the short axis of the opening has a length B. The ball head is mounted inside the spherical chamber and has a diameter C, in which B < A ≤ C.

The artificial hip joint prosthesis according to embodiments of the present invention prevents the ball head from detaching from the inner liner and improves the safety of using the hip joint prosthesis.

In some embodiments, an outer surface of the ball head and an inner surface of the spherical chamber are spherical surfaces that fit with each other.

In some embodiments, the ball head has a first hole, the hole forms a circular section on the ball head, and a height of the ball head in a direction perpendicular to the circular section is D, in which D ≤ B < C.

In some embodiments, the artificial hip joint prosthesis further includes a connector. A first end of the connector extends into the first hole, and the first hole of the ball head is connected with a cone head of the connector; and a second end of the connector is connected with another prosthesis.

In some embodiments, a ball height in an axial direction of the first hole during insertion of the ball head is smaller or equal to the length of the short axis of the elliptical opening of the inner liner.

In some embodiments, a detachment force detaching the ball head from the spherical chamber is F1, and a press-in force pressing the ball head into the spherical chamber is F2, in which 2F2 ≤ F1.

In some embodiments, the opening of the spherical chamber includes an elliptical shape and a quasi-elliptical shape.

In some embodiments, the inner spherical surface of the spherical chamber adopts materials including polyethylene, ceramic or metal.

In some embodiments, the ball head adopts materials including metal or ceramic.

In another aspect, embodiments of the present invention provide a method of for mounting an artificial hip joint prosthesis that is the artificial hip joint prosthesis according to any one of the above embodiments. The method includes: aligning or paralleling the axis of the first hole with the short axis of the opening, and mounting the ball head into the spherical chamber; and rotating the ball head by 90° and making the first hole face an exterior of the chamber, after the ball head is in the spherical chamber.

### BRIEF DESCRIPTION OF DRAWINGS

Fig. 1 is an exploded view of a hip joint double-acting head prosthesis according to an embodiment of the present invention.
Fig. 2 is a top view of a hip joint double-acting head prosthesis according to an embodiment of the present invention.
Fig. 3 is a sectional view of a hip joint double-acting head prosthesis according to an embodiment of the present invention.
Fig. 4 shows a diagram of a comparative experiment between press-in/detachment force of a hip joint double-acting head prosthesis according to an embodiment of the present invention and press-in/detachment force of an existing hip joint prosthesis in the related art.

### Reference numerals:

outer shell 1, first accommodation cavity 11,
inner liner 2, chamber 21, opening 211,
ball head 3, first hole 31.

### DETAILED DESCRIPTION

Embodiments of the present invention will be described in detail and examples of the embodiments will be shown in the accompanying drawings. The embodiments described below with reference to the accompanying drawings are exemplary and intended to explain the present invention rather than limit the present invention.

An artificial hip joint prosthesis according to embodiments of the present invention is composed of a spherical chamber and a ball head. An outer spherical surface of the ball head fits with an inner spherical surface of the chamber to form a spherical hinge. The inner spherical surface of the chamber has an opening in an elliptical shape or a similar shape with a long axis and a short axis, to allow insertion of the ball head. The long axis of the opening in the elliptical shape or the similar shape has a length denoted as A, and the short axis of the opening has a length denoted as B. The ball head is mounted inside the chamber and has a diameter denoted as C, in which B < A ≤ C.

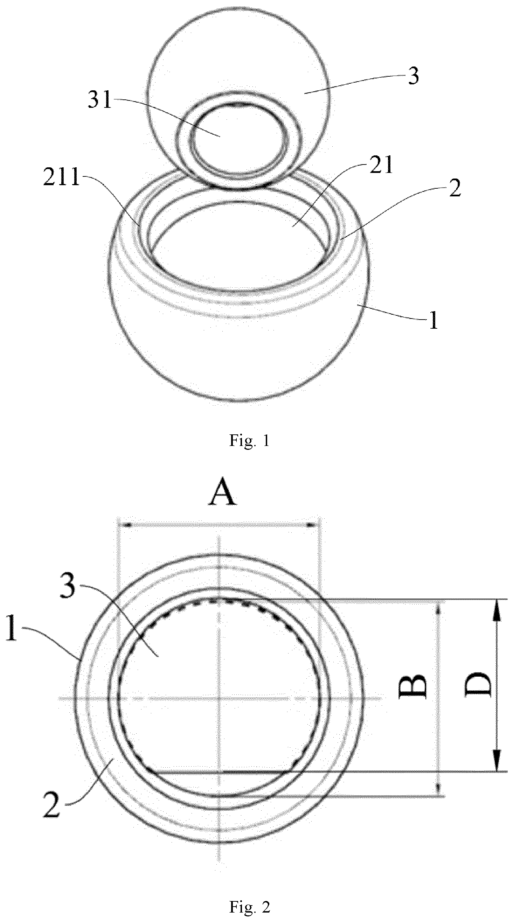

In some embodiments, as shown in Fig. 1, includes an outer shell 1, an inner liner 2 and a ball head 3. The outer shell 1 has a first accommodation cavity 11, and the inner liner 2 is arranged in the first accommodation cavity 11. The inner liner 2 has a chamber 21 with an opening 211 on a side of the chamber 21 away from the outer shell 1. The ball head 3 is mounted into and clamped in the chamber 21. A spherical outer wall surface of the ball head 3 is in contact with a spherical inner wall surface of the inner liner 2, to form a spherical hinge. A diameter of the ball head 3 is larger than or equal to a long axis of the elliptical opening 211.

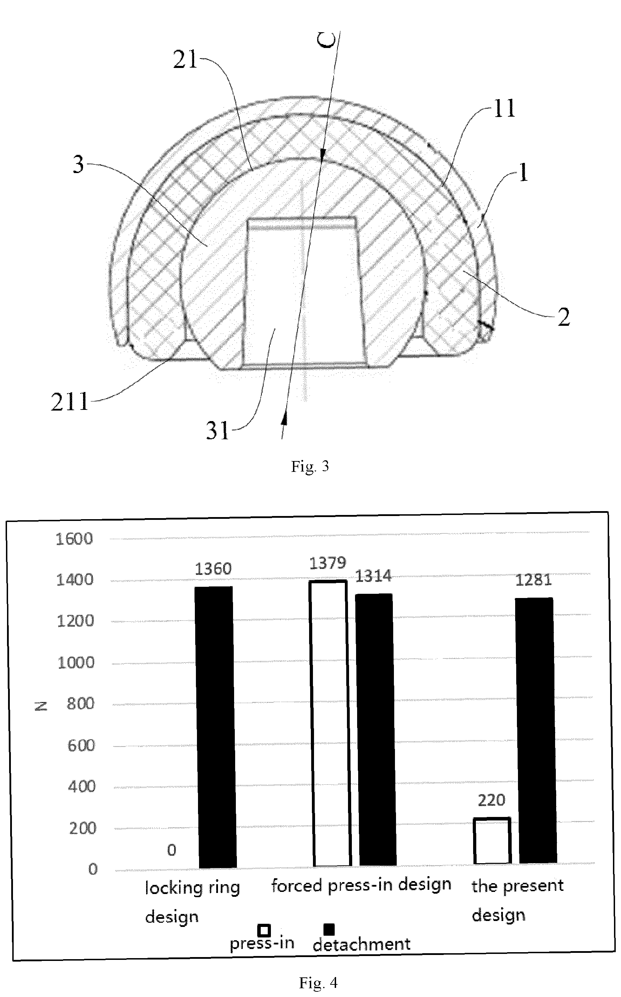

In some embodiments, as shown in Figs. 1 and 3, the outer shell 1 is wrapped outside the inner liner 2, and an outer wall surface of the inner liner 2 is in locking contact with an inner wall surface of the outer shell 1 and fixed in the first accommodation cavity 11. A spherical outer wall surface of the outer shell 1 is in direct contact with a human acetabulum. The spherical outer wall surface of the ball head 3 is in contact with the spherical inner wall surface of the inner liner 2, and the spherical hinge is formed between the ball head 3 and the inner liner 2, that is, the ball head 3 can rotate 360 degrees inside the inner liner 2.

As shown in Fig. 2, for example, the long axis of the elliptical opening 211 has a length denoted as A, a short axis of the elliptical opening 211 has a length denoted as B, and the diameter of the ball head 3 is denoted as C.

For example, the length of the long axis of the elliptical opening 211 is equal to or slightly smaller than the diameter of the ball head 3, while the length of the short axis of the elliptical opening 211 is significantly smaller than the diameter of the ball head 3, in which B < A ≤ C.

In some embodiments, an outer surface of the ball head and an inner surface of the chamber are spherical surfaces that fit with each other.

In some embodiments, the ball head has a first hole that forms a circular section on the ball head. A height of the ball head in a direction perpendicular to the circular section is D, in which D ≤ B < C.

As shown in Figs. 2 and 3, the ball head includes a first hole 31 that forms a circular section on the ball head. The height of the circular section is D, in which D < B < A ≤ C. When the ball head is mounted in the chamber 21, an axis of the first hole of the ball head 3 is parallel to the short axis of the elliptical opening 211, causing minimum interference between the ball head and the elliptical opening 211, and releasing a press-in force. After the ball head 3 is mounted in the chamber 21, the ball head 3 rotates 90 degrees to make the first hole 31 face outwards and return to a working position. It can be ensured that the ball head 3 cannot come out after returning to the working position since the length of the opening 211 of the inner liner 2 in all directions is smaller than the diameter of the ball head 3. Meanwhile, it can be ensured that the first hole always faces outwards after the first hole of the ball head is mounted in a femoral stem cone (not shown), and the ball head is locked from returning to a position at the start of installation, thus avoiding a risk of joint dislocation.

In some embodiments, the human hip joint prosthesis further includes a connector (not shown). A first end of the connector extends into the first hole 31 of the ball head, and a cone head of the connector is connected with the first hole 31. A second end of the connector is configured to be connected with another prosthesis.

In some embodiments, a ball height in an axial direction of the first hole 31 during insertion of the ball head 3 is smaller or equal to the length of the short axis of the elliptical opening 211 of the inner liner 2, so that resistance against the insertion is small. After insertion, the ball head is rotated by 90 degrees to return to the working position, so that a contact surface between the ball head 3 and the elliptical opening 211 of the inner liner 2 is restored to a complete circle, and a force that detaches the ball head 3 from the inner liner 2 is maximized.

In some embodiments, a detachment force that detaches the ball head 3 from the chamber is denoted as F1, and a press-in force that presses the ball head 3 into the chamber is denoted as F2, in which 2F2 ≤ F1.

In some embodiments, the opening 211 of the chamber includes not only an elliptical shape, but also a quasi-elliptical shape.

In some embodiments, the spherical surface of the chamber adopts materials such as polyethylene, ceramic or metal.

In some embodiments, the material of the ball head 3 is metal or ceramic.

A process of assembling the hip joint prosthesis according to embodiments of the present invention will be described below with reference to Figs. 1 to 3.

During insertion of the ball head 3 into the chamber 21, the axis of the first hole 31 of the ball head 3 is parallel to the short axis of the elliptical opening 211. At this time, an orientation of the first hole 31 of the ball head 3 is orthogonal to an orientation of the elliptical opening 211 of the chamber 21. The ball head 3 is pressed downwards, and hence an edge of the inner liner 2 is elastically deformed to generate resistance. The ball head 3 is pressed into the chamber 21 while a pressing force of the ball head 3 is greater than the resistance. Since pressure on the ball head in a direction along the short axis of the opening 211 of the inner liner 2 is released, and the length of the long axis is equal to or slightly smaller than the diameter of the ball head 3, resistance in a direction along the long axis is limited, and the resistance on the ball head 3 during installation is reduced. The ball head 3 is rotated by 90 degrees after the ball head 3 is mounted into the chamber 21. In such a way, the orientation of the first hole 31 at a lower end of the ball head 3 is identical to the orientation of the elliptical opening 211 of the chamber 21. Since the length of the opening 211 of the inner liner 2 in all directions is smaller than the diameter of the ball head 3, it can be ensured that the ball head 3 cannot come out after being pressed back to the working position, thus avoiding the risk of joint dislocation.

Fig. 4 shows a comparative experiment between press-in/detachment force of the design according to the present invention and press-in/detachment force of two existing designs (i.e., forced press-in and a locking ring). The experiment result shows that under the premise of reaching a detachment force of 1300 N like the two existing designs, a press-in force of the design according to the present invention is only 220 N, which is 1/6 of the detachment force. However, the press-in force of the design adopting forced press-in is even higher than the detachment force. It can be seen that the design according to the present invention has a very remarkable effect. The experiment also shows that downward pressure exerted by an ordinary adult with bare hands is at least 400 N, so that doctors can easily press the ball head into the inner liner with bare hands during surgery under the condition of obtaining sufficient anti-detachment force, which greatly facilitates the surgical process.

Embodiments of the present invention provide a method for mounting an artificial hip joint prosthesis that is the artificial hip joint prosthesis according to any of the above embodiments. The method includes: aligning or paralleling the axis of the first hole with the short axis of the opening; mounting the ball head into the chamber; and after the ball head is in the chamber, rotating the ball head by 90° and making the first hole face an exterior of the chamber.

In the specification, it is to be understood that terms such as "central," "longitudinal," "transverse," "length," "width," "thickness," "upper," "lower," "front," "rear," "left," "right," "vertical," "horizontal," "top," "bottom," "inner," "outer," "clockwise," "counterclockwise," "axial," "radial," "circumferential" and the like should be construed to refer to the orientation as then described or as shown in the drawings under discussion. These relative terms are for convenience and simplicity of description and do not indicate or imply that the devices or elements referred to must have a specific orientation or be constructed and operated in a specific orientation. These terms cannot be construed as limitation on the present invention.

In addition, terms such as "first" and "second" are used herein for purposes of description and are not intended to indicate or imply relative importance or the number of indicated technical features. Therefore, features defined as "first" and "second" can explicitly or implicitly include one or more of this feature. In the description of the present invention, the term "a plurality of" means at least two, such as two or three, unless specified otherwise.

In the present invention, unless specified or limited otherwise, terms "mounted," "connected," "coupled," "fixed" and the like are used broadly, and may be, for example, fixed connections, detachable connections, or integral connections; may also be mechanical or electrical connections; may also communicate with each other; may also be direct connections or indirect connections via intervening structures; may also be inner connection or mutual interaction of two elements, which can be understood by those skilled in the art according to specific situations.

In the present invention, unless specified or limited otherwise, a structure in which a first feature is "on" or "below" a second feature may include an embodiment in which the first feature is in direct contact with the second feature, and may also include an embodiment in which the first feature and the second feature are not in direct contact with each other, but are contacted via an additional feature formed therebetween. Furthermore, a first feature "on," "above," or "on top of" a second feature may include an embodiment in which the first feature is right or obliquely "on," "above," or "on top of" the second feature, or just means that the first feature is at a height higher than that of the second feature; while a first feature "below," "under," or "on bottom of" a second feature may include an embodiment in which the first feature is right or obliquely "below," "under," or "on bottom of" the second feature, or just means that the first feature is at a height lower than that of the second feature.

Reference throughout this specification to "an embodiment," "some embodiments," "an example," "a specific example," or "some examples," means that a particular feature, structure, material, or characteristic described in connection with the embodiment or example is included in at least one embodiment or example of the present invention. Thus, the appearances of these phrases in various places throughout this specification are not necessarily referring to the same embodiment or example of the present invention. Furthermore, the particular features, structures, materials, or characteristics may be combined in any suitable manner in one or more embodiments or examples. Moreover, those skilled in the art may combine and unite different embodiments or examples described in this specification, as well as features of different embodiments or examples, without conflicting with each other.

Although embodiments of the present invention have been shown and described, it would be appreciated by those skilled in the art that the above embodiments are exemplary and cannot be construed as limitation on the present invention, and changes, modifications, alternatives and variations can be made in the embodiments without departing from the scope of the present invention.

## Claims

1. An artificial hip joint prosthesis, comprising a spherical chamber and a ball head,
wherein an outer spherical surface of the ball head fits with an inner spherical surface of the spherical chamber to form a spherical hinge;
an inner spherical surface of the spherical chamber has an opening in an elliptical shape or a similar shape with a long axis and a short axis, to allow insertion of the ball head;
the long axis of the opening in the elliptical shape or the similar shape has a length A, and the short axis of the opening has a length B; and
the ball head is mounted inside the spherical chamber and has a diameter C, wherein B < A ≤ C.

2. The artificial hip joint prosthesis according to claim 1, wherein an outer surface of the ball head and an inner surface of the spherical chamber are spherical surfaces that fit with each other.

3. The artificial hip joint prosthesis according to claim 1 or 2, wherein the ball head has a first hole, the hole forms a circular section on the ball head, and a height of the ball head in a direction perpendicular to the circular section is D, wherein D ≤ B < C.

4. The artificial hip joint prosthesis according to claim 3, further comprising a connector,
wherein a first end of the connector extends into the first hole, and the first hole of the ball head is connected with a cone head of the connector; and a second end of the connector is connected with another prosthesis.

5. The artificial hip joint prosthesis according to claim 3 or 4, wherein a ball height in an axial direction of the first hole during insertion of the ball head is smaller or equal to the length of the short axis of the elliptical opening of the inner liner.

6. The artificial hip joint prosthesis according to any one of claims 1 to 5, wherein a detachment force detaching the ball head from the spherical chamber is F1, and a press-in force pressing the ball head into the spherical chamber is F2, wherein 2F2 ≤ F1.

7. The artificial hip joint prosthesis according to any one of claims 1 to 6, wherein the opening of the spherical chamber comprises an elliptical shape and a quasi-elliptical shape.

8. The artificial hip joint prosthesis according to any one of claims 1 to 7, wherein the inner spherical surface of the spherical chamber adopts materials comprising polyethylene, ceramic or metal.

9. The artificial hip joint prosthesis according to any one of claims 1 to 8, wherein the ball head adopts materials comprising metal or ceramic.

10. A method for mounting an artificial hip joint prosthesis according to any one of claims 1 to 9, comprising:
aligning or paralleling the axis of the first hole with the short axis of the opening, and mounting the ball head into the spherical chamber; and
rotating the ball head by 90° and making the first hole face an exterior of the chamber, after the ball head is in the spherical chamber.
